# EUROPEAN PATENT SPECIFICATION

(11) **EP 4 091 564 B1**
(45) Date of publication and mention of the grant of the patent: **17.12.2025**
(21) Application number: 22173660.6
(22) Date of filing: 17.05.2022
(51) Int. Cl.: A61B 18/14, A61B 18/00, A61B 18/18, A61B 18/24, A61B 17/00

(54) **IMPROVING EFFICIENCY OF IRE ABLATION PROCEDURE BY APPLYING STRESS SIGNAL TO TARGET TISSUE**
VERBESSERUNG DER EFFIZIENZ EINES IRE-ABLATIONSVERFAHRENS DURCH ANLEGEN EINES SPANNUNGSSIGNALS AN DAS ZIELGEWEBE
AMÉLIORATION DE L'EFFICACITÉ D'UNE PROCÉDURE D'ABLATION D'ÉLECTROPORATION IRRÉVERSIBLE PAR APPLICATION D'UN SIGNAL DE STRESS AU TISSU CIBLE

(30) Priority: 18.05.2021 US 202117323112
(43) Date of publication of application: 23.11.2022
(73) Proprietor: Biosense Webster (Israel) Ltd, Yokneam, 2066717 (IL)
(72) Inventor: ALTMANN, Andres Claudio, 2066717 Yokneam (IL); GOVARI, Assaf, 2066717 Yokneam (IL)
(74) Representative: Carpmaels & Ransford LLP

(56) References cited:
- US-A1- 2007 062 547
- US-A1- 2019 175 248
- US-A1- 2021 106 249

## Description

### FIELD OF THE INVENTION

The present invention relates generally to medical devices, and particularly to systems for improving efficiency of irreversible electroporation (IRE) ablation procedures.

### BACKGROUND OF THE INVENTION

Various techniques for performing irreversible electroporation (IRE) procedures and other sorts of cell-killing procedures have been published.

For example, U.S. Patent Application Publication No. 2020/0316376 describes systems, methods, and apparatus for electroporation, which may include an applicator; an endoscope, trocar or the like; a generator; and a drug delivery device. The applicator may include a control portion, an insertion tube connected to the control portion, an actuator engaged with the control portion, and a plurality of electrodes comprising a first electrode having a first tip and a second electrode having a second tip. The plurality electrodes may be configured to move between a retracted position and a deployed position in response to actuation by the actuator.

U.S. Patent Application Publication No. 2012/0085649 describes devices and methods for performing dielectrophoresis. The devices contain a sample channel which is separated by physical barriers from electrode channels which receive electrodes. The devices and methods may be used for the separation and analysis of particles in solution, including the separation and isolation of cells of a specific type. As the electrodes do not make
contact with the sample, electrode fouling is avoided and sample integrity is better maintained. US 2021/106249 discloses a system for lesion formation assessment in tissue that has undergone one or more ablation procedures. A medical device is used to thermally and/or energetically treat the targeted area of tissue area to create at least one ablation lesion once baseline impedance measurements have been recorded, i.e. to ablate the area of tissue. The impedance sensing electrode(s) are used to assess or record a second series of one or more post-treatment impedance characteristics over time upon completion of the treatment procedure. The post-treatment impedance measurements may include impedance characteristics taken with similar parameters to the baseline impedance measurements. The post-treatment impedance measurements may be taken after the delivery of a low dose of electroporation energy and/or after the delivery of a high dose of electroporation energy.

### SUMMARY OF THE INVENTION

The invention is defined in claim 1. An embodiment of the present invention that is describe herein provides a system including first and second circuitries and one or more devices. The first circuitry is configured to generate a stress signal for reducing an impedance of tissue of an organ. The second circuitry is configured to generate an irreversible electroporation (IRE) signal for producing a lesion in the tissue. The one or more devices are configured to apply to the tissue, the stress signal at a first time interval, and the IRE signal at a second time interval, subsequent to the first time interval.

In some embodiments, the one or more devices include one or more electrodes, which are coupled to a catheter inserted into the organ and are configured to apply the stress signal and the IRE signal from the catheter. In other embodiments, the one or more devices include one or more first electrodes, which are coupled to a first catheter inserted into the organ and are configured to apply the stress signal, and one or more second electrodes, which are coupled to a second catheter inserted into the organ and are configured to apply the IRE signal. In yet other embodiments, the organ includes a heart, the stress signal includes a radiofrequency (RF) pulse having a power larger than 10 watts, and the first time interval is between 0.1 second and 1 second.

In an embodiment, the first circuitry and the second circuitry include a common pulse generator, which is configured to generate: (i) the stress signal in a first pulse including a radiofrequency (RF) pulse having a power larger than 10 watts, and having the first time interval
between 0.1 second and 1 second, and (ii) the IRE signal in a second pulse, different from the first pulse, and including a processor, which is configured to control the common pulse generator to apply the second pulse within less than 30 seconds after applying the first pulse. In another embodiment, the first circuitry includes an ultrasound generator, the stress signal includes an ultrasound signal produced by the ultrasound generator, and at least one of the one or more devices includes an ultrasound transducer configured to apply the ultrasound signal to the tissue.

In some embodiments, the first circuitry includes a light source, the stress signal includes a light beam produced by the light source, and at least one of the one or more devices includes an optical device configured to apply the light beam to the tissue. In other embodiments, the light source includes a laser and the light beam includes a laser beam. In yet other embodiment, the first circuitry includes a microwave generator, the stress signal includes a microwave signal produced by the microwave generator, and at least one of the one or more devices includes a microwave antenna configured to apply the microwave signal to the tissue.

### BRIEF DESCRIPTION OF THE DRAWINGS

The present invention will be more fully understood from the following detailed description of the embodiments thereof, taken together with the drawings in which:
Fig. 1 is a schematic, pictorial illustration of a catheter-based position-tracking and irreversible electroporation (IRE) ablation system, in accordance with an exemplary embodiment of the present invention; and
Fig. 2 is a flow chart that schematically illustrates an unclaimed method for improving efficiency of an IRE ablation procedure by applying a stress signal prior to an IRE ablation signal.

### DETAILED DESCRIPTION OF EMBODIMENTS

### OVERVIEW

Irreversible electroporation (IRE), also referred to herein as IRE ablation, may be used, for example, for treating arrhythmia by ablating tissue cells using high-voltage applied pulses. Cellular destruction occurs when the transmembrane potential exceeds a threshold, leading to cell death and formation of a lesion.

The demand for IRE ablations is growing rapidly. Such ablation procedures; however, can typically be carried out only by physicians specializing in electrophysiology, and consume resources that are in short supply such as operating rooms. It is therefore advantageous to improve the efficiency of such procedures.

Exemplary embodiments of the present invention that are described hereinbelow provide techniques for improving the efficiency of ablation procedures, and more specifically, improving the efficiency of IRE ablations.

In principle, the duration of IRE ablation could be reduced by increasing the power (e.g., voltage) applied to the tissue in question. However, applying excess voltage may be hazardous to the patient safety and typically requires expensive equipment (e.g., a high power IRE pulse generator).

In some embodiments, a system for improving efficiency of IRE ablation comprises first circuitry, which is configured to generate a stress signal for reducing the impedance of tissue intended to subsequently receive one or more IRE ablation signals. The system comprises second circuitry, which is configured to generate the IRE ablation signal for producing a lesion in the tissue. In the context of the present disclosure and in the claims, the terms "IRE signal" and "IRE ablation signal" are used interchangeably and refer to the irreversible electroporation signals applied to the tissue in question (e.g., heart tissue or tissue of any other suitable organ) for treating arrhythmia or another medical disease.

In some embodiments, the system comprises one or more devices, which are configured to apply to the tissue in question, the stress signal at a first time interval, and the IRE signal at a second time interval, subsequent to the first time interval. Note that for a given IRE signal (e.g., a voltage pulse) applied to the tissue in question, reducing the tissue impedance (prior to applying the IRE signal) increases the current passing through the tissue, increases the voltage on the cell membrane, and consequently, increases the size of the lesion formed by applying the IRE signal.

In the context of the present disclosure and in the claims, the terms "tissue intended to be ablated," "tissue intended to receive the IRE signal" and "tissue in question" are used interchangeably and refer to cells of tissue, which is targeted to be killed and transformed into a lesion in order to treat arrhythmia in the patient organ (e.g., patient heart).

In some embodiments, the one or more devices comprise one or more electrodes, which are coupled to a catheter inserted into the patient heart and are configured to apply the stress signal and the IRE signal from the catheter.

In other embodiments, the one or more devices comprise one or more first electrodes, which are coupled to a first catheter inserted into the heart and are configured to apply the stress signal, and one or more second electrodes, which are coupled to a second different catheter inserted into the heart, and are configured to apply the IRE signal.

In some embodiments, the stress signal may comprise a radiofrequency voltage pulse, for example, a pulse having power larger than about 10 watts, and the duration of the first time interval is between about 0.1 second and 1 second. In the context of the present disclosure and in the claims, the terms "about" or "approximately" for any numerical values or ranges indicate a suitable dimensional tolerance that allows the part or collection of components to function for its intended purpose as described herein.

In other embodiments, the system may comprise another sort of apparatus or circuitry configured to generate the stress signal using any other suitable type of energy source, and the one or more devices are configured to apply the stress signal to the tissue in question. For example, the circuitry that is configured to generate the stress signal, may comprise an ultrasound generator, or a laser source, or a microwave generator, or any other suitable type of circuitry. In such examples, the stress signal may comprise an ultrasound signal, or a laser beam, or a microwave signal, which are produced by the ultrasound generator, the laser source, and the microwave generator, respectively. Moreover, the aforementioned one or more devices may comprise one or more ultrasound transducers, or optical devices, or microwave antennas, respectively, which are coupled to a catheter inserted into the patient heart and that are configured to apply the respective stress signal to the tissue in question.

By improving the ablation efficiency and reducing the amount of power required to be applied to the tissue in question for producing the required size of lesion, the disclosed techniques improve the patient safety and reduce the cost of irreversible electroporation procedures, and other sorts of ablation procedures.

### SYSTEM DESCRIPTION

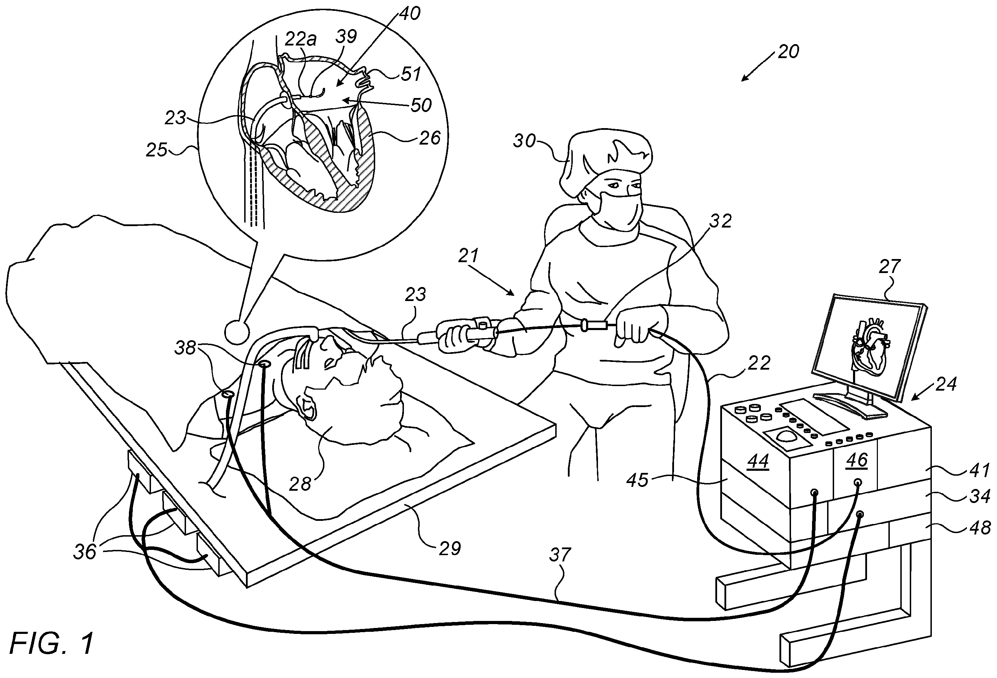

Fig. 1 is a schematic, pictorial illustration of a catheter-based position-tracking and irreversible electroporation (IRE) ablation system 20, in accordance with an exemplary embodiment of the present invention.

Reference is now made to an inset 25. In some embodiments, system 20 comprises a deflectable tip section 40 that is fitted at a distal end 22a of a shaft 22 of a catheter 21 with deflectable tip section 40 comprising multiple electrodes 50.

In the embodiment described herein, electrodes 50 are configured to apply IRE signals to tissue of a heart 26, in the present example, to carry out IRE ablation procedure in the left atrium of a heart 26, such as IRE ablation of an ostium 51 of a pulmonary vein (PV) in heart 26. In some embodiments, one or more of electrodes 50 are further configured to sense intra-cardiac (IC) electrocardiogram (ECG) signals in heart 26. Note that the techniques disclosed herein are applicable, *mutatis mutandis,* to other sections (e.g., atrium or ventricle) of heart 26, and to other organs of a patient 28.

Reference is now made back to the general view of Fig. 1. In some embodiments, the proximal end of catheter 21 is connected to a control console 24 (also referred to herein as a console 24, for brevity) comprising an ablative power source, in the present example an IRE pulse generator (IPG) 45, which is configured to deliver peak power in the range of tens of kilowatts (kWs). Console 24 comprises a switching box 46, which is configured to switch the power applied by IPG 45 to one or more selected pairs of electrodes 50. A sequenced IRE ablation protocol may be stored in a memory 48 of console 24.

In some embodiments, IPG 45 is configured to generate one or more IRE signal(s), which may comprise any suitable combination of a sequence of IRE pulses, also referred to herein as IRE pulse trains, defined in a protocol of a unipolar or bipolar IRE ablation stored in a memory of console 24 and controlled, for example, by a processor 41 described below. Methods and systems for generating and applying protocols of IRE pulse trains to tissue in question are described in detail, for example, in U.S. Patent Application Nos. 17/234,625 and 16/993, 092.

In some embodiments, a physician 30 inserts distal end 22a of shaft 22 through a sheath 23 into heart 26 of patient 28 lying on a table 29. Physician 30 navigates distal end 22a of shaft 22 to a target location in heart 26 by manipulating shaft 22 using a manipulator 32 positioned near the proximal end of catheter 21. During the insertion of distal end 22a, deflectable tip section 40 is maintained in a straightened configuration by sheath 23. By containing tip section 40 in a straightened configuration, sheath 23 also serves to minimize vascular trauma when physician 30 moves catheter 21, through the vasculature of patient 28, to the target location, such as an ablation site, in heart 26.

In some embodiments, once distal end 22a of shaft 22 has reached the ablation site, physician 30 retracts sheath 23 and deflects tip section 40, and further manipulates shaft 22 to place electrodes 50 disposed over tip section 40 in contact with ostium 51 at the ablation site. In the present example, the ablation site comprises one or more PVs of heart 26, but in other embodiments, physician 30 may select any other suitable ablation site.

In some embodiments, electrodes 50 are connected by wires running through shaft 22 to processor 41, which is configured to control several components of system 20, such as switching box 46 using interface circuits 44 of console 24.

As further shown in inset 25, distal end 22a comprises a position sensor 39 of a position tracking system, which is coupled to distal end 22a, e.g., at tip section 40. In the present example, position sensor 39 comprises a magnetic position sensor, but in other embodiments, any other suitable type of position sensor (e.g., other than magnetic-based) may be used. During navigation of distal end 22a in heart 26, processor 41 receives signals from magnetic position sensor 39 in response to magnetic fields from external field generators 36, for example, for the purpose of measuring the position of tip section 40 in heart 26 and, optionally, for displaying the tracked position overlaid on the image of heart 26, on a display 27 of console 24. Magnetic field generators 36 are placed at known positions external to patient 28, e.g., below table 29. Console 24 also comprises a driver circuit 34, configured to drive magnetic field generators 36.

The method of position sensing using external magnetic fields is implemented in various medical applications, for example, in the CARTO^{™} system, produced by Biosense Webster Inc. (Irvine, Calif.) and is described in detail in U.S. Patent Nos. 5,391,199, 6,690,963, 6,484,118, 6,239,724, 6,618,612 and 6,332,089, in PCT Patent Publication WO 96/05768, and in U.S. Patent Application Publication Nos. 2002/0065455 A1, 2003/0120150 A1 and 2004/0068178 A1.

Typically, processor 41 of console 24 comprises a general-purpose processor of a general-purpose computer, with suitable front end and interface circuits 44 for receiving signals from catheter 21, as well as for applying ablation energy via catheter 21 in a left atrium of heart 26 and for controlling the other components of system 20. Processor 41 typically comprises a software in memory 48 of system 20, which is programmed to carry out the functions described herein. The software may be downloaded to the computer in electronic form, over a network, for example, or it may, alternatively or additionally, be provided and/or stored on non-transitory tangible media, such as magnetic, optical, or electronic memory.

### PERFORMING IRREVERSIBLE ELECTROPORATION ABLATION DURING HEART REFRACTORY PERIOD

Irreversible electroporation (IRE), also referred to as Pulsed Field Ablation (PFA), may be used as a minimally invasive therapeutic modality to kill tissue cells at the ablation site by applying high-voltage pulses to the tissue. In the present example, IRE pulses may be used for killing myocardium tissue cells in order to treat cardiac arrhythmia in heart 26. Cellular destruction occurs when the transmembrane potential exceeds a threshold, leading to cell death and thus the development of a tissue lesion. Therefore, of particular interest is the use of high-voltage bipolar electrical pulses, e.g., using a pair of electrodes 50 in contact with tissue at the ablation site, to generate high electric fields (e.g., above a certain threshold) to kill tissue cells located between the electrodes.

In the context of this disclosure, "bipolar" voltage pulse refers to a voltage pulse applied between two electrodes 50 of catheter 21. The bipolar voltage pulses differ, for example, from unipolar pulses that are applied, e.g., during a radiofrequency (RF) ablation, by a catheter electrode relative to some common ground electrode not located on the catheter.

The demand for IRE ablations is growing rapidly. Such ablation procedures, however, can typically be carried out only by physicians specializing in electrophysiology, and consume resources that are in short supply such as operating rooms. It is therefore advantageous to reduce the cycle time of such procedures. In principle, the duration of IRE ablation could be reduced by increasing the power (e.g., voltage) applied to the tissue in question. However, applying excess power may be hazardous to the patient safety and typically requires expensive equipment (e.g., a high power IRE pulse generator).

In some embodiments, system 20 comprises circuitry, which is configured to generate a stress signal for reducing the impedance of tissue intended to subsequently receive one or more IRE ablation signals. In the present example, the circuitry may comprise IPG 45, which is further configured to apply one or more stress signals comprising RF pulses having a power larger than about 10 watts. More specifically, IPG 45 is configured to apply RF pulses having a power of about 90 watts for a duration between about 0.1 seconds and 1 second (e.g., about 0.5 second). The duration of applying the RF pulses of the stress signal is also referred to herein as a first time interval.

In some embodiments, the stress signal, which is applied during the first time interval, prior to applying the IRE ablation signal results in reducing the impedance of the tissue in question. Thus, for a given IRE signal (e.g., a voltage pulse) applied to the tissue in question, reducing the tissue impedance (prior to applying the IRE signal) increases the current passing through the tissue, increases the voltage on the cell membrane, and consequently increases the size of the lesion formed by applying the IRE signal. In other words, applying an IRE signal to tissue that has already received the stress signal, typically results in a larger (e.g., deeper and/or wider) lesion formed in the tissue, compared to applying the IRE signal with no preceding stress signal. Alternatively, in order to obtain a lesion having a given depth, physician 30 may control system 20 to: (i) apply a given voltage-over-time of the IRE signal to tissue that received no stress signal in advance, or (ii) apply a lower power (e.g., lower voltage and/or shorter duration) of the IRE signal to tissue that has already received the aforementioned stress signal.

In embodiments in which a stress signal is applied, processor 41 is configured to apply to tissue that has already received the stress signal, a given IRE voltage for a shorter duration, compared to the duration of an IRE signal that has the same voltage and is applied to tissue that did not receive the stress signal during the first time interval. In other words, applying the stress signal to the tissue in question improves the efficiency of the IRE signal subsequently applied to the same tissue. Note that in order to improve the efficiency of the IRE signal applied to the tissue in question, it is important to apply the IRE signal between a few second and about 30 seconds after applying the stress signal. In other words, a shorter delay between the stress and IRE signals, increases the efficiency of the IRE signal. In such embodiments, processor 41 is configured to control IPG 45 to: (i) apply the stress signal at the first time interval, and (ii) apply the IRE signal at a second time interval within less than about 30 seconds after applying the stress signal in the first time interval.

For example, in heart tissue that received at the first time interval, an RF stress signal of about 90 watts for a duration of about 0.5 second, a lesion having a size of about 5 mm may be formed by applying, in the second subsequent time interval of about 0.25 seconds, an IRE signal having a voltage of about 2000 volts between electrodes having an inter-electrode distance of about 10 mm. A similar heart tissue that did not receive the stress signal in advance, may be treated with an IRE signal having the same 2000 volts and about 10 mm inter-electrode distanced, applied to the tissue for about 0.25 seconds, so as to obtain a smaller lesion having a lesion size of about 3.5 mm. In other words, when applying to tissue a stress signal less than about 30 seconds prior to applying the same conditions of IRE signal, results in an increased size of the lesion by about 1.4 times (e.g., from about 3.5 mm to about 5 mm).

In some embodiments, processor 41 is configured to apply both the stress signal and the IRE signal to the heart tissue using the same set of electrodes 50 during the first and second time intervals, respectively.

In other embodiments, processor 41 is configured to apply the stress signal to a first subset of electrodes 50, and to apply the IRE signal to a second, different, subset of electrodes 50 of catheter 21. Additionally or alternatively, catheter 21 may comprise more than one type of electrodes, also referred to herein as first and second type of electrodes. In this example embodiment, the first type of electrode(s) may be used to apply the stress signal, and the second type of electrode(s) may be used to apply the IRE signal.

In yet other embodiments, system 20 may comprise an additional catheter (not shown), which is inserted into heart 26 and is placed in contact with the tissue intended to receive the IRE ablation signal. The additional catheter has one or more given electrodes (not shown) configured to apply the stress signal and/or the IRE signal to the tissue in question. In such embodiments, processor 41 is configured to apply the stress signal to the given electrodes, and to apply the IRE signal to electrodes 50. Alternatively, processor 41 may apply the stress signal to electrodes 50 and the IRE signal to the given electrodes.

In other embodiments, system 20 may comprise one or more surface electrodes 38, coupled to the skin of patient 28 and attached by wires running through a cable 37 to the chest and shoulder of patient 28. In such embodiments, the stress signal is applied to the tissue by applying the voltage, e.g., between one surface electrode 38 and one of the given electrodes described above.

In alternative embodiments, system 20 may comprise another sort of apparatus or circuitry configured to generate the stress signal using any other suitable type of energy source, and one or more devices (other than electrodes 50), which are coupled to a catheter inserted into heart 26, and are configured to apply the other type of stress signal to the tissue in question. For example, the circuitry that is configured to generate the stress signal, may comprise an ultrasound generator (not shown). In this example, the stress signal may comprise an ultrasound signal, which is produced by the ultrasound generator. Moreover, the aforementioned devices may comprise one or more ultrasound transducers, which are coupled to a catheter inserted into heart 26 and that are configured to apply the ultrasound signal to the tissue in question.

In other embodiments, the circuitry that is configured to generate the stress signal may comprise any suitable type of a light source (not shown), e.g., a laser source. In such embodiments, the stress signal may comprise a light beam such as a laser beam, which is produced by the laser source. In such embodiments, at least one of the one or more devices may comprise an optical device, which is coupled to a catheter inserted into heart 26 and is configured to apply the laser beam to the tissue in question.

In yet other embodiments, the circuitry that is configured to generate the stress signal may comprise any suitable type of a microwave generator (not shown). In such embodiments, the stress signal may comprise a microwave signal, which is produced by the microwave generator, and at least one of the one or more devices comprises at least a microwave antenna, which is coupled to a catheter

inserted into heart 26 and is configured to apply the microwave signal to the tissue in question.

In other embodiments, surface electrodes 38 are configured to sense body-surface (BS) ECG signals in response to beats of heart 26. Acquisition of BS ECG signals may be carried out using conductive pads attached to the body surface or any other suitable technique, and the acquired BS ECG signals may be used for determining the IRE ablation signal applied to the tissue in question.

### IMPROVING IRE ABLATION PROCEDURE BY APPLYING TO TARGET TISSUE STRESS SIGNAL PRIOR TO IRE ABLATION SIGNAL

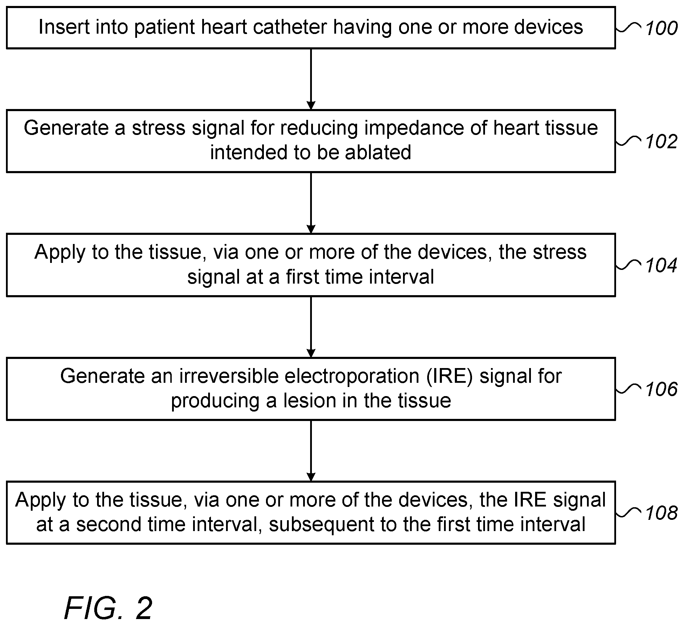

Fig. 2 is a flow chart that schematically illustrates an unclaimed method for improving efficiency of an IRE ablation procedure by applying to tissue of heart 26 a stress signal prior to an IRE ablation signal.

The method begins at a catheter insertion step 100, with inserting into patient heart 26 one or more catheters, such as catheter 21, having one or more devices coupled to tip section 40. In the present example, the one or more devices comprise electrodes 50 shown in Fig. 1 above. In other embodiments, at least one of the devices may comprise another type of electrode coupled to tip section 40, or another device, such as an ultrasound transducer, an optical device, or a microwave antenna, as described in detail in Fig. 1 above. In some embodiments, the devices may be coupled to a single catheter or to multiple different catheters.

At a stress signal generation step 102, processor 41 controls IPG 45 to generate a stress signal for reducing impedance of the tissue intended to be ablated in heart 26. In the present example the stress signal comprises an RF voltage pulse having a power of about 90 watts. In other embodiments, system 20 comprises another type of apparatus or circuitry configured to generate a different sort of stress signal. For example, an ultrasound generator, a laser source, and a microwave generator, each of which configured to generate a stress signal, which is intended to reduce the impedance of the tissue in question, as described in detail in Fig. 1 above.

At a stress signal application step 104, processor 41 controls IPG 45 (or any other suitable type of circuitry or apparatus, as described in Fig. 1 above) to apply the stress signal generated in step 102 above, via the aforementioned one or more devices, to the tissue in question of heart 26 during a first time interval. In the present example, the duration of the first time interval is about 0.5 second. In other embodiments, the first time interval may have any other suitable duration, for example, between 0.1 second and 1 second.

In some embodiments, the one or more of the devices are coupled to the catheter inserted into heart 26. For example, the stress signal is applied to the tissue via one or more electrodes 50 coupled to tip section 40, or via other sort of electrodes or devices, such as but not limited to the ultrasound transducer, the optical device, and the microwave antenna coupled to catheter 21 or to an additional catheter inserted into heart 26.

At an irreversible electroporation signal generation step 106, processor 41 controls IPG 45 to generate an IRE signal for producing a lesion in the tissue in question of heart 26. In some embodiments, the IRE signal may comprises any suitable combination of IRE pulse trains defined in a protocol of a unipolar or bipolar IRE ablation stored in and controlled by processor 41, as described in Fig. 1 above.

At an IRE signal application step 108 that concludes the unclaimed method, processor 41 controls IPG 45 to apply the IRE signal to the tissue in question, via electrodes 50 coupled to tip section 40, or via any other of the aforementioned devices that may be coupled to catheter 21 or to an additional catheter inserted into heart 26.

In some embodiments, the IRE signal is applied to the tissue in question at a second time interval, which is subsequent to the first time interval in which the same tissue had already received the stress signal during the first time interval, as described in step 104. Note that after applying the stress signal, the impedance of the tissue is reduced, and therefore, the power and/or duration of the IRE signal applied to the same tissue, may be reduced, as described in detail in Fig. 1 above.

In some embodiments, both steps 102 and 106 may be carried out using IPG 45 that can generate both the stress signal and the IRE signal. Moreover, both steps 104 and 108 may be carried out by electrodes 50 that can apply both the stress signal and the IRE signal to the tissue intended to be ablated. In an embodiment, the same electrodes 50 may be used for applying both signals at the first and second time intervals. In another embodiment, a first subset of electrodes 50 may be used for applying the stress signal at the first time interval, and a second subset of electrodes 50 may be used for applying the IRE signal at the second subsequent time interval. In this embodiment, the first and second subsets may or may not have one or more common electrodes.

In other embodiments, step 102 may be carried out using circuitry or apparatus other than IPG 45, such as but not limited to the ultrasound generator, the laser source, or the microwave generator described in Fig. 1 above. In such embodiments, step 104 may be carried out using one or more devices other than electrodes 50, which may be coupled to catheter 21 or to any other catheter inserted into heart 26. The devices are typically corresponding to the type of stress signal and the generator thereof. For example, (i) an ultrasound transducer coupled to the catheter for applying the ultrasound-based stress signal produced by the ultrasound generator, (ii) an optical device coupled to the catheter for applying the laser beam-based stress signal produced by the laser source, or (iii) a microwave antenna coupled to the catheter for applying the microwave-based stress signal produced by the microwave generator. In such embodiments, steps 104 and 108 are carried out using IPG 45 and electrodes 50 as described above.

Although the embodiments described herein mainly address irreversible electroporation for treating arrhythmia in patient heart, the methods and systems described herein can also be used in other applications, such as in treatment against cancer in various organs, such as but not limited to liver and lungs.

## Claims

1. A system for improving IRE ablation efficiency, the system comprising:
first circuitry, which is configured to generate a stress signal for reducing an impedance of tissue of an organ;
second circuitry, which is configured to generate an irreversible electroporation (IRE) signal for producing a lesion in the tissue; and
one or more devices, which are configured to apply to the tissue, the stress signal at a first time interval, and the IRE signal at a second time interval, subsequent to the first time interval.

2. The system according to claim 1, wherein the one or more devices comprise one or more electrodes, which are coupled to a catheter inserted into the organ and are configured to apply the stress signal and the IRE signal from the catheter.

3. The system according to claim 1, wherein the one or more devices comprise one or more first electrodes, which are coupled to a first catheter inserted into the organ and are configured to apply the stress signal, and one or more second electrodes, which are coupled to a second catheter inserted into the organ and are configured to apply the IRE signal.

4. The system according to claim 1, wherein the organ comprises a heart, wherein the stress signal comprises a radiofrequency (RF) pulse having a power larger than 10 watts, and wherein the first time interval is between 0.1 second and 1 second.

5. The system according to claim 1, wherein the first circuitry and the second circuitry comprise a common pulse generator, which is configured to generate: (i) the stress signal in a first pulse comprising a radiofrequency (RF) pulse having a power larger than 10 watts, and having the first time interval between 0.1 second and 1 second, and (ii) the IRE signal in a second pulse, different from the first pulse, and comprising a processor, which is configured to control the common pulse generator to apply the second pulse within less than 30 seconds after applying the first pulse.

6. The system according to claim 1, wherein the first circuitry comprises an ultrasound generator, wherein the stress signal comprises an ultrasound signal produced by the ultrasound generator, and wherein at least one of the one or more devices comprises an ultrasound transducer configured to apply the ultrasound signal to the tissue.

7. The system according to claim 1, wherein the first circuitry comprises a light source, wherein the stress signal comprises a light beam produced by the light source, and wherein at least one of the one or more devices comprises an optical device configured to apply the light beam to the tissue.

8. The system according to claim 7, wherein the light source comprises a laser and wherein the light beam comprises a laser beam.

9. The system according to claim 1, wherein the first circuitry comprises a microwave generator, wherein the stress signal comprises a microwave signal produced by the microwave generator, and wherein at least one of the one or more devices comprises a microwave antenna configured to apply the microwave signal to the tissue.

## Patentansprüche

1. System zum Verbessern einer IRE-Ablationseffizienz, das System umfassend:
eine erste Schaltlogik, die konfiguriert ist, um ein Spannungssignal zum Reduzieren einer Impedanz eines Gewebes eines Organs zu erzeugen;
eine zweite Schaltlogik, die konfiguriert ist, um ein irreversibles Elektroporationssignal (IRE-Signal) zum Herstellen einer Läsion in dem Gewebe zu erzeugen; und
eine oder mehrere Vorrichtungen, die konfiguriert sind, um auf dem Gewebe das Spannungssignal in einem ersten Zeitintervall und das IRE-Signal in einem zweiten Zeitintervall nach dem ersten Zeitintervall anzuwenden.

2. System nach Anspruch 1, wobei die eine oder die mehreren Vorrichtungen eine oder mehrere Elektroden umfassen, die mit einem in das Organ eingeführten Katheter gekoppelt sind und konfiguriert sind, um das Spannungssignal und das IRE-Signal aus dem Katheter anzuwenden.

3. System nach Anspruch 1, wobei die eine oder die mehreren Vorrichtungen eine oder mehrere erste Elektroden umfassen, die mit einem ersten in das Organ eingeführten Katheter gekoppelt sind und konfiguriert sind, um das Spannungssignal anzuwenden, und eine oder mehrere zweite Elektroden, die mit einem zweiten in das Organ eingeführten Katheter gekoppelt sind und konfiguriert sind, um das IRE-Signal anzuwenden.

4. System nach Anspruch 1, wobei das Organ ein Herz umfasst, wobei das Spannungssignal einen Hochfrequenzimpuls (HF-Impuls) mit einer Leistung von mehr als 10 Watt umfasst, und wobei das erste Zeitintervall zwischen 0,1 Sekunde und 1 Sekunde liegt.

5. System nach Anspruch 1, wobei die erste Schaltlogik und die zweite Schaltlogik einen gemeinsamen Impulsgenerator umfassen, der konfiguriert ist, um zu erzeugen: (i) das Spannungssignal in einem ersten Impuls, umfassend einen Hochfrequenzimpuls (HF-Impuls) mit einer Leistung von mehr als 10 Watt und dessen erstes Zeitintervall zwischen 0,1 Sekunde und 1 Sekunde liegt, und (ii) das IRE-Signal in einem zweiten Impuls, der sich von dem ersten Impuls unterscheidet, und umfassend einen Prozessor, der konfiguriert ist, um den gemeinsamen Impulsgenerator zu steuern, um den zweiten Impuls innerhalb von weniger als 30 Sekunden nach dem Anlegen des ersten Impulses anzulegen.

6. System nach Anspruch 1, wobei die erste Schaltlogik einen Ultraschallgenerator umfasst, wobei das Spannungssignal ein von dem Ultraschallgenerator erzeugtes Ultraschallsignal umfasst, und wobei mindestens eine der einen oder der mehreren Vorrichtungen einen Ultraschallwandler umfasst, der konfiguriert ist, um das Ultraschallsignal auf das Gewebe anzuwenden.

7. System nach Anspruch 1, wobei die erste Schaltlogik eine Lichtquelle umfasst, wobei das Spannungssignal einen von der Lichtquelle erzeugten Lichtstrahl umfasst, und wobei mindestens eine der einen oder der mehreren Vorrichtungen eine optische Vorrichtung umfasst, die konfiguriert ist, um den Lichtstrahl auf das Gewebe anzuwenden.

8. System nach Anspruch 7, wobei die Lichtquelle einen Laser umfasst und wobei der Lichtstrahl einen Laserstrahl umfasst.

9. System nach Anspruch 1, wobei die erste Schaltlogik einen Mikrowellengenerator umfasst, wobei das Spannungssignal ein von dem Mikrowellengenerator erzeugtes Mikrowellensignal umfasst, und wobei mindestens eine der einen oder der mehreren Vorrichtungen eine Mikrowellenantenne umfasst, die konfiguriert ist, um das Mikrowellensignal auf das Gewebe anzuwenden.

## Revendications

1. Système permettant d'améliorer une efficacité d'ablation IRE, le système comprenant :
un premier système de circuits, qui est configuré pour générer un signal de contrainte permettant de réduire une impédance de tissu d'un organe ;
un second système de circuits, qui est configuré pour générer un signal d'électroporation irréversible (IRE) permettant de produire une lésion dans le tissu ; et
un ou plusieurs dispositifs, qui sont configurés pour appliquer au tissu, le signal de contrainte à un premier intervalle de temps, et le signal IRE à un second intervalle de temps, postérieur au premier intervalle de temps.

2. Système selon la revendication 1, dans lequel le ou les dispositifs comprennent une ou plusieurs électrodes, qui sont accouplées à un cathéter inséré dans l'organe et sont configurées pour appliquer le signal de contrainte et le signal IRE à partir du cathéter.

3. Système selon la revendication 1, dans lequel le ou les dispositifs comprennent une ou plusieurs premières électrodes, qui sont accouplées à un premier cathéter inséré dans l'organe et sont configurées pour appliquer le signal de contrainte, et une ou plusieurs secondes électrodes, qui sont accouplées à un second cathéter inséré dans l'organe et sont configurées pour appliquer le signal IRE.

4. Système selon la revendication 1, dans lequel l'organe comprend un cœur, dans lequel le signal de contrainte comprend une impulsion de radiofréquence (RF) ayant une puissance plus grande que 10 watts, et dans lequel le premier intervalle de temps est compris entre 0,1 seconde et 1 seconde.

5. Système selon la revendication 1, dans lequel le premier système de circuits et le second système de circuits comprennent un générateur d'impulsions commun, qui est configuré pour générer : (i) le signal de contrainte dans une première impulsion comprenant une impulsion de radiofréquence (RF) ayant une puissance plus grande que 10 watts, et ayant le premier intervalle de temps entre 0,1 seconde et 1 seconde, et (ii) le signal IRE dans une seconde impulsion, différente de la première impulsion, et comprenant un processeur, qui est configuré pour commander le générateur d'impulsions commun pour appliquer la seconde impulsion moins de 30 secondes après l'application de la première impulsion.

6. Système selon la revendication 1, dans lequel le premier système de circuits comprend un générateur d'ultrasons, dans lequel le signal de contrainte comprend un signal d'ultrasons produit par le générateur d'ultrasons, et dans lequel au moins l'un parmi le ou les dispositifs comprend un transducteur à ultrasons configuré pour appliquer le signal d'ultrasons au tissu.

7. Système selon la revendication 1, dans lequel le premier système de circuits comprend une source de lumière, dans lequel le signal de contrainte comprend un faisceau de lumière produit par la source de lumière, et dans lequel au moins l'un parmi le ou les dispositifs comprend un dispositif optique configuré pour appliquer le faisceau de lumière au tissu.

8. Système selon la revendication 7, dans lequel la source de lumière comprend un laser et dans lequel le faisceau de lumière comprend un faisceau laser.

9. Système selon la revendication 1, dans lequel le premier système de circuits comprend un générateur de micro-ondes, dans lequel le signal de contrainte comprend un signal de micro-ondes produit par le générateur de micro-ondes, et dans lequel au moins une parmi le ou les dispositifs comprend une antenne à micro-ondes configurée pour appliquer le signal de micro-ondes au tissu.
